(19) **Europäisches Patentamt**

**European Patent Office**

**Office européen des brevets**

(11) **EP 1 259 825 B1**

(12) **EUROPÄISCHE PATENTSCHRIFT**

(45) Veröffentlichungstag und Bekanntmachung des Hinweises auf die Patenterteilung:
**11.05.2005 Patentblatt 2005/19**

(21) Anmeldenummer: **01909783.1**

(22) Anmeldetag: **20.02.2001**

(51) Int Cl.7: **G01N 33/78**, G01N 33/76

(86) Internationale Anmeldenummer:
**PCT/EP2001/001905**

(87) Internationale Veröffentlichungsnummer:
**WO 2001/063296 (30.08.2001 Gazette 2001/35)**

(54) **VERFAHREN ZUR DIFFERENTIALDIAGNOSTISCHEN BESTIMMUNG VON GEGEN DEN TSH-REZEPTOR GEBILDETEN AUTOANTIKÖRPERN IN EINER SERUM- ODER PLASMAPROBE EINES PATIENTEN**

METHOD FOR CARRYING OUT THE DIFFERENTIAL DIAGNOSTIC DETERMINATION OF AUTOANTIBODIES FORMED AGAINST THE TSH RECEPTOR IN A SERUM SAMPLE OR PLASMA SAMPLE OF A PATIENT

PROCEDE POUR DETERMINER, PAR UN DIAGNOSTIC DIFFERENTIEL, LA PRESENCE D'AUTO-ANTICORPS FORMES CONTRE LE RECEPTEUR TSH DANS UN ECHANTILLON DE SERUM OU DE PLASMA D'UN PATIENT

(84) Benannte Vertragsstaaten:
**DE FR IT**

(30) Priorität: **21.02.2000 DE 10007792**

(43) Veröffentlichungstag der Anmeldung:
**27.11.2002 Patentblatt 2002/48**

(73) Patentinhaber: **B.R.A.H.M.S Aktiengesellschaft**
**16761 Hennigsdorf (DE)**

(72) Erfinder:
  • **BERGMANN, Andreas**
    **12351 Berlin (DE)**
  • **LOOS, Ulrich**
    **89081 Ulm (DE)**
  • **MINICH, Waldemar**
    **13589 Berlin (DE)**

(74) Vertreter: **Andrae, Steffen, Dr. et al**
**Andrae Flach Haug**
**Balanstrasse 55**
**81541 München (DE)**

(56) Entgegenhaltungen:
**WO-A-98/20343**

• **MINICH W.B., LOOS U.: "Detection of functionally different types of pathological autoantibodies against thyrotropin receptor in Graves' patients sera by luminescent immunoprecipitation analysis" EXP CLIN ENDOCRINOL DIABETES, Nr. 108, April 2000 (2000-04), Seiten 110-119, XP000993378**
• **NAGAYAMA Y. ET AL: "Binding domains of stimulatory and inhibitory thyrotropin (TSH) receptor autoantibodies determined with chimeric TSH-lutropin/chorionic gonadotropin receptors" THE JOURNAL OF CLINICAL INVESTIGATION, Bd. 88, Juli 1991 (1991-07), Seiten 336-340, XP002062530**
• **GRASSO Y.Z. ET AL: "Epitope heterogeneity of thyrotropin receptor blocking antibodies in Graves' patients as detected with wild-type versus chimeric thyrotropin receptors" THRYROID, Bd. 9, Nr. 6, 1999, Seiten 531-537, XP001013584 in der Anmeldung erwähnt**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 259 825 B1

**Beschreibung**

Die vorliegende Erfindung betrifft ein Verfahren zur differentialdiagnostischen, selektiven Bestimmung von gegen den TSH-Rezeptor gebildeten Autoantikörpern in einer Serum- oder Plasmaprobe eines Patienten.

Der Rezeptor für das Hormon TSH (thyroidstimulierendes Hormon), der TSH-Rezeptor (TSHR), spielt eine Schlüsselrolle für die Funktion und das Wachstum von Schilddrüsenzellen. Dieser Rezeptor ist ein Glied einer Unterfamilie der G-Protein-gekoppelten Glykoproteinrezeptoren, die außerdem insbesondere noch die Rezeptoren für das luteinisierende Hormon/Choriongonadotrophin (LH/CGR) und das follikelstimulierende Hormon (FSHR) umfaßt. Die Rezeptoren dieser Unterfamilie weisen eine große N-terminale extrazelluläre Domäne auf, die für die Ligandenbindung von essentieller Bedeutung ist und für die gezeigt wurde, daß sie an der Signalübermittlung beteiligt ist. Die Übermittlung des TSHR-Signals wird überwiegend durch die Aktivierung von Adenylatcyclase vermittelt, was zu einer Erhöhung des intrazellulären cAMP-Niveaus führt.

Ein Teil des großen Interesses an dem TSHR ist auf seine Rolle als primäres Autoantigen bei verschiedenen Schilddrüsen-Autoimmunerkrankungen zurückzuführen, die vom Auftreten von Autoantikörpern gegen den TSHR begleitet sind (TSHR-Auto-Ab). Zu derartigen Schilddrüsen-Autoimmunerkrankungen gehören insbesondere die Basedowsche Krankheit (Morbus Basedow; engl.: Graves' disease), eine zu Schilddrüsenüberfunktion führende Autoimmunerkrankung, die zu den häufigsten menschlichen Autoimmunerkrankungen überhaupt gehört, sowie die Hashimoto Thyreoiditis und das idiopathische Myxödem, die mit einer Schilddrüsenunterfunktion verbunden sein können.

Der TSHR kommt in der Oberfläche von Schilddrüsenzellen nur in sehr geringen Mengen (in der Größenordnung von 1.000 - 10.000 Rezeptoren pro Zelle) vor. Eine Aufklärung der chemischen Struktur des humanen TSH-Rezeptors (hTSHR), d.h. der Sequenz der für ihn codierenden DNA sowie der daraus ableitbaren Aminosäuresequenz des Rezeptors selbst, gelang Ende 1989 (vgl. Libert F. et al., Biochem. Biophys. Res. Commun. 165: 1250-1255; Nagayama Y. et al., Biochem. Biophys. Res. Commun. 165: 1184-1190; vgl. auch EP-A-0433509 bzw. WO-A-91/09121; sowie WO-A-91/09137; WO-A-91/10735 und WO-A-91/03483; ferner Yuji Nagayama & Basil Rapoport, in: Molecular Endocrinology, Vol. 6 No. 2, S. 145-156 und die darin zitierte Literatur). Seitdem wurden das hTSHR-Polypeptid und ausgewählte Teilpeptide davon in einer Vielzahl von Systemen exprimiert, wobei sich zeigte, daß ein funktionaler TSHR nur in Säugetierzellen hergestellt werden kann, und zwar sowohl transient als auch in Form einer stabilen Zellinie, z. B. unter Verwendung von Adenovirus- und Vacciniavirus-Expressionssystemen.

Die traditionelle Schwierigkeit, den hTSHR ohne Funktionalitätsverlust zu markieren und in einer löslichen bzw. dispergierbaren Form bereitzustellen, erschwerte bis heute die Entwicklung von klinisch einsetzbaren direkten Immunpräzipitationsassays zum Nachweis von pathologischen hTSHR-Autoantikörpern.

Aus der Arbeit von N.G.Morgenthaler at al., Exp Clin Endocrinol Diabetes 104 (1996) Suppl 4, S.56-59 ist zwar ein Immunpräzipitationsassay für TSHR-Auto-Ab bekannt. Bei diesem Assay wird als Reagens zur Fällung der in einer Probe vorhandenen TSHR-Auto-Ab eine Präparation eines extrazellulären Teils eines rekombinanten, durch Einbau von $^{35}$S-Methionin markierten TSH-Rezeptors verwendet. Der Assay weist weder eine Selektivität für stimulierende (TSAb), noch für blockierende (TBAb) TSHR-Auto-Ab auf, erfaßt jedoch auch solche TSHR-Auto-Ab, die nicht mit bTSH kompetieren. Die Herstellung des $^{35}$S-markierten Rezeptors durch in vitro-Translation ist jedoch außerordentlich aufwendig und teuer, und es gibt keine Meßgeräte, die für eine klinische Routinemessung der von $^{35}$S emittierten Strahlung geeignet sind. Das Verfahren ist daher als Meßverfahren für die klinische Routinediagnostik nicht geeignet.

Aus DE 198 01 154 bzw. J. Endocrinol. 160:239-245 (1999) ist ein Verfahren bekannt, das es ermöglicht, einen vollständigen funktionalen rhTSHR in reiner, solubilisierter und radioaktiv markierter Form herzustellen. Es wird u.a. vorgeschlagen, einen solchen radioaktiv markierten rhTSHR zur Bestimmung der Gesamtmenge an TSHR-Auto-Ab in einem Präzipitationsassay zu verwenden, der als Vorteil aufweist, daß er nicht nur solche TSHR-Auto-Ab erfaßt, die mit markiertem TSH kompetieren (d.h. sog. "die TSH-Bindung inhibierende Immunglobuline" oder TBII). Das vorgeschlagene Verfahren gestattet es nicht, innerhalb der in einer Probe vorhandenen TSHR-Auto-Ab eine Unterscheidung nach stimulierenden (TSAb), blockierenden (TBAb) sowie ggf. weder stimulierenden noch blockierenden, "neutralen" TSHR-Auto-Ab zu treffen. Außerdem ist ein Arbeiten mit einem radioaktiven Label in vielen Fällen grundsätzlich unerwünscht.

Das Verfahren gemäß DE 198 01 154 baut auf auf einem Verfahren zur Herstellung von funktionalen rhTSHR-Fusionsprodukten, das in DE 196 45 729 bzw. WO 98/20343 beschrieben wurde und dort zur Herstellung von rhTSHR-Fusionsproteinen diente, die zusätzlich zur TSHR-Sequenz relativ kurze Peptidreste für eine Immobilisierung bzw. Markierung des TSHR bzw. einen biotinylierten Peptidrest aufwiesen. Die Herstellung von Fusionsproteinen mit langen Proteinresten, z.B. Enzymresten, wurde noch nicht berichtet. Da auf die in DE 196 45 729 und DE 198 01 154 offenbarten Arbeitsweisen auch im Rahmen der vorliegenden Anmeldung zurückgegriffen wird, wird auf den Inhalt der beiden Veröffentlichungen zur Ergänzung der Offenbarung der vorliegenden Anmeldung ausdrücklich Bezug genommen.

Der einzige gegenwärtig in der klinischen Praxis verwendbare Assay zur Bestimmung von TSHR-Auto-Ab ist

EP 1 259 825 B1

daher ein indirekt arbeitender kompetitiver Assay, bei dem die Inhibierung der Bindung von markiertem bTSH an einen solubilisierten porcinen TSHR aus Schweine-Schilddrüsen oder an einen für "Coated Tube" Anwendungen immobilisierten rhTSHR gemäß DE 196 51 093 oder WO 98/26294 bzw. DE 198 01 319 oder WO 99/3678 bzw. J.Clin.Endocinol. Metab., Vol.84, No.1, S.90-97 (1999) zur Messung genutzt wird. Er erfaßt nur die Autoantikörper vom sog. TBII-Typ.

Da inzwischen bekannt ist, daß TSHR-Auto-Ab auch im Falle von Morbus Basedow Patienten nicht nur stimulierende Autoantikörper, die die Hyperthyreose hervorrufen, umfassen, sondern daneben auch noch nicht-pathogene "neutrale" Autoantikörper und sogar blockierende, wäre es allerdings wünschenswert, auch einen Assay zur Verfügung zu haben, der für einen zu bestimmenden Typ von Autoantikörpern selektiv ist und z.B. eine Unterscheidung von pathogenen stimulierenden und blockierenden und "neutralen" Autoantikörpern ermöglicht.

Zur Unterscheidung von stimulierenden, blockierenden und neutralen Autoantikörpern wurden bisher im wesentlichen Bioassays genutzt, bei denen ermittelt wird, ob die cAMP-Produktion von Zellen, die in ihrer Membran einen natürlichen oder rekombinanten TSHR enthalten, durch die Anwesenheit von bestimmten Seren bzw. Autoantikörpern erhöht wird bzw. ob die durch eine gegebene TSH-Konzentration in der Probe bewirkte cAMP-Produktion durch die Seren oder Autoantikörper geschwächt wird. Im Rahmen derartiger Untersuchungen bzw. zur Ermittlung derjenigen Bereiche der Sequenz der extrazellulären Domäne des TSHR, an die TSH bzw. die verschiedenen Typen von Autoantikörpern (TSAb, TBAb) binden, wurde dabei auch schon mit hTSHR-Chimären gearbeitet, bei denen bestimmte hTSHR-Teilsequenzen durch entsprechende Sequenzen anderer G-Protein-gekoppelter Rezeptoren, und zwar insbesondere von Ratten-LH-CG-Rezeptoren, ersetzt waren. Dabei zeigte sich in cAMP-Stimulationsassays unter Verwendung von Zellen, die diese Chimären exprimieren, daß für die Bindung von stimulierenden TSHR-Auto-Ab (TSAb) offensichtlich die Aminosäuren des N-Terminus des hTSHR benötigt werden, und zwar insbesondere Aminosäuren der Sequenz 8-165, gerechnet vom Methionin-Anfangsrest. Für die Bindung von blockierenden TSHR-Auto-Ab (TBAb) dagegen erwiesen sich Aminosäuren vom C-Terminus der extrazellulären Domäne des hTHSR, insbesondere Aminosäuren der Sequenz 261-370, als erforderlich.

Im Zusammenhang mit der Verwendung von TSHR/LHCGR-Chimären in Bioassays sind insbesondere zu nennen die Arbeiten von Tahara K, Ban K, Minegishi T, Kohn LD. 1991, Biochem Biophys Res Commun. 179: 70-77; Tahara K, Ishikawa N, Yamamoto K, Hirai A, Ito K, Tamura Y, Yoshida S, Saito Y, Kohn LD. 1997, Thyroid 7: 867-877 und Grasso YZ, Kim MR, Falman C, Kohn LD, Tahara K, Gupta MK. 1999, Thyroid 9: 531-537. Auf die in den genannten Arbeiten zitierten weiteren Literaturstellen wird zur Vertiefung verwiesen.

Es ist Aufgabe der vorliegenden Erfindung, ein neues differentialdiagnostisches Verfahren zur selektiven Bestimmung von TSHR-Auto-Ab mit unterschiedlicher pathogener Wirkung zu schaffen.

Diese Aufgabe wird durch ein neues selektives Verfahren zur Bestimmung von stimulierenden bzw. blockierenden TSHR-Auto-Antikörpern gemäß Anspruch 1 gelöst.

Vorteilhafte, gegenwärtig bevorzugte Ausgestaltungen des erfindungsgemäßen Verfahrens werden in den Unteransprüchen 2 bis 5.

Im vorausgehenden und nachfolgenden Text dieser Anmeldung werden die verwendeten Reagenzien bzw. Analyten/Biomoleküle in der Regel durch verschiedene Abkürzungen gekennzeichnet, die stets in den folgenden Bedeutungen zu verstehen sind, es sei denn, es ergibt sich für den Fachmann aus dem konkreten Zusammenhang etwas anderes. Die Verwendung der speziellen Angaben erfolgt dabei aus Gründen der exakten Beschreibung der durchgeführten Versuche und Messungen, bedeutet jedoch nicht, daß die beschriebenen Ergebnisse und Schlußfolgerungen nur für den beschriebenen Spezialfall gelten und nicht unter Heranziehung des relevanten Fachwissens verallgemeinert werden können.

| | |
|---|---|
| TSH = | Thyroidstimulierendes Hormon (Thyreotropin). Wird die Abkürzung TSH ohne weitere Zusätze verwendet, handelt es sich nicht um ein bestimmtes Produkt, sondern die Bindung bzw. Funktion des Hormons wird in allgemeiner Form diskutiert. |
| $^{125}$I-bTSH = | Radioiodiertes, als Kompetitor eingesetztes bTSH (bovines TSH). Im Zusammenhang mit der Beschreibung von Assays der Firma B.R.A.H.M.S Diagnostica GmbH steht $^{125}$I-bTSH insbesondere für ein Produkt, wie es gemäß DE 42 37 430 C1 bzw. EP 0 668 775 B1 erhalten wird und Bestandteil des TRAK-Assay® der Fa. B.R.A.H.M.S Diagnostica GmbH ist. |
| TSHR = | Der TSH-Rezeptor, ein in der Schilddrüsenmembran verankerter Glykoproteinrezeptor. Wird die Abkürzung TSHR ohne weitere Zusätze verwendet, handelt es sich nicht um ein bestimmtes Produkt, sondern die Funktion des Rezeptors bzw. seine Bindungsbeteiligung wird in allgemeiner Form diskutiert. |
| rTSHR = | Gentechnisch erzeugtes (rekombinantes) Polypeptid, das die Aminosäuresequenz eines natürlich vorkommenden TSHR, insbesondere eines humanen TSHR (hTSHR), wenigstens |

in einem solchen Ausmaße aufweist, daß es als "funktionaler TSH-Rezeptor" bezeichnet werden kann, was bedeutet, daß es sich bezüglich der Bindung von Autoantikörpern gegen den TSHR bzw. von hTSH in signifikantem Ausmaß wie der natürlich vorkommende, insbesondere humane TSHR verhält. Wird die Abkürzung rTSHR ohne weitere Zusätze verwendet, handelt es sich nicht um ein bestimmtes Produkt, d.h. rTSHR kann für das rekombinante vollständige, mehr oder weniger glykosylierte Polypeptid, eine Teilsequenz einer ausreichenden Länge oder ein gentechnologisch erzeugtes Fusionsprodukt davon stehen (wie es z.B. in der Internationalen Patentanmeldung PCT/EP97/06121 beschrieben wird). Ohne weitere Erläuterungen liegt ein einfach als "rTSHR" oder "rhTSHR" bezeichnetes Produkt als Membranpräparation vor, aus der das Produkt ggf. durch geeignete Solubilisierung von Membranen der zur Expression des rekombinanten Polypeptids verwendeten Zellen unter Verwendung von Detergenzien auch als aufgereinigter Extrakt erhalten wurde.

| | |
|---|---|
| WT rTSHR = | "Wildtyp TSHR" - ein im wesentlichen vollständiger rhTSHR zur Bestimmung der Gesamtmenge an TSHR-Auto-Ab (Total) in einer Probe. |
| Fusions-TSHR = | rhTSHR mit angefügten Aminosäuresequenzen, die die Polypeptidsequenz eines funktionalen TSHR verlängern. |
| TSHR-LUC = | Um den Proteinrest eines Enzyms, und zwar in allen Beispielen eines Leuchtkäfer-Luciferase-Enzyms (LUC), verlängertes rhTSHR-Fusionsprotein. |
| TSHR/LH-CGR = | "TSHR-Chimäre", d.h. ein rhTSHR, bei dem Teile der Aminosäuresequenz durch vergleichbare Sequenzen eines anderen Rezeptors mit einem anderem Bindungsverhalten gegenüber TSHR-Auto-Ab, insbesondere nichtbindenden Sequenzen, und zwar gemäß allen Beispielen durch Sequenzen eines Ratten LH-CG-Rezeptors (LH-CGR), ersetzt sind. Die verschiedenen konkrte verwendeten TSHR-Chimären werden als Chimären A, B und C (oder auch "Wildtyp-Chimären" WT-A, WT-B und WT-C) näher charakterisiert. |
| TSHR/LH-CGR-LUC = | rhTSHR-Fusionsprotein, das eine TSHR-Chimäre sowie einen Enzymrest enthält, der in allen Beispielen ein Leuchtkäfer-Luciferase-Rest ist. |
| TSHR-Auto-Ab = | In biologischen Proben, insbesondere humanem Serum oder Plasma, nachweisbare Autoantikörper gegen den TSH-Rezeptor beliebiger Spezifität. |
| TSAb = | Die Schilddrüse stimulierende TSHR-Auto-Ab. Ihr Nachweis ist insbesondere für die Diagnose des Morbus Basedow (englisch: Graves' disease) von Bedeutung. |
| TBAb = | Die Wirkung von TSH auf die Schilddrüse blockierende TSHR-Auto-Ab, deren Nachweis zu einer Hypothyreose in Beziehung gesetzt werden kann. |
| TNAb = | TSHR-Auto-Ab, die weder zu den TSAb noch den TBAb gehören. |
| TBII = | In kompetitiven Assays, die auf einer Konkurrenz von markiertem TSH mit TSHR-Auto-Ab um die Bindungsstellen einer TSHR-Präparation beruhen, erfaßbare TSHR-Auto-Ab. Sie können TSAb und/oder TBAb und/oder neutrale TSHR-Auto-Ab sein. Ein kommerzieller Assay (Radiorezeptorassay) zur Bestimmung von TSHR-Auto-Ab vom TBII-Typ ist der TRAK-Assay® der Fa. B.R.A.H.M.S Diagnostica GmbH. |

Im Rahmen der Arbeiten, die in der vorliegenden Anmeldung beschrieben werden, wurden mit der Absicht, selektive Immunpräzipitationsassays zur Bestimmung von TSHR-Auto-Ab-Typen zu schaffen, neue Reagenzien in Form von Fusionsproteinen geschaffen, die einen Leuchtkäfer-Luciferase-Rest als Beispiel für einen detektierbaren Enzymrest enthalten, sowie einen rekombinanten funktionalen humanen TSH-Rezeptor (rhTSHR), der auch als "Wildtyp-TSHR" (WT TSHR) bezeichnet wird, sowie drei verschiedene Chimären des Wildtyp-TSHR (Chimären A, B und C). Die Chimären wurden so konstruiert, daß aufgrund von Erkenntnissen, die mit Bioassays unter Verwendung derartiger Chimären gewonnen worden waren, die Hoffnung bestand, daß sie für stimulierende oder blockierende TSHR-Auto-Ab selektiv sein könnten. Eine weitere Chimäre (Chimäre C) war eine solche, die weder mit stimulierenden noch blockierenden TSHR-Auto-Ab reagieren sollte und daher für sogenannte "neutrale" Autoantikörper (TNAb) ohne bekannte pathogene Wirkung selektiv sein müßte, wenn Seren tatsächlich auch derartige neutrale TSHR-Auto-Ab enthalten.

Mit den genannten neuen Reagenzien wurden verschiedene Versuchsreihen durchgeführt, in denen festgestellt werden sollte, ob die Anfügung eines Enzym-Proteinrests die Funktionalität des TSHR-Rests im Fusionsprodukt beeinträchtigt und/oder ob durch die Einbindung in ein Fusionsprodukt die Enzymaktivität des angefügten Enzymrests signifikant verändert wird. Derartige Untersuchungen waren auch deshalb erforderlich, weil die bisher bekannten TSHR-Fusionsprodukte stets nur kürzere Peptidreste, jedoch keine langen Enzymreste von proteinischer Natur enthielten.

Wie nachfolgend genauer gezeigt wird, erwies es sich, daß die Funktionalität des TSHR bzw. der TSHR-Chimären durch die Anfügung eines Enzymrests nicht signifikant verändert wird, und daß außerdem auch der Enzymrest, der in den konkreten Beispielen ein Leuchtkäfer-Luciferase-Rest war, seine Wirkung behält und für die übliche Nachweisreaktion verwendet werden kann.

Nachdem man festgestellt hatte, daß die TSHR- bzw. TSHR-Chimären-Enzym-Fusionsprodukte die gewünschten Reaktivitäten aufwiesen, wurden sie in Immunpräzipitationsassays eingesetzt, wobei Seren von Normalpersonen und Patienten mit Schilddrüsenerkrankungen getestet wurden und die erhaltenen Ergebnisse zu den Ergebnissen in Beziehung gesetzt wurden, die mit den vorbekannten Assays erhalten wurden, nämlich einmal mit einem kompetitiven Assay zur Bestimmung von TSHR-Auto-Ab, die mit TSH kompetieren (TBII), und zum anderen mit Bioassays, bei denen die cAMP-Produktion von transformierten Zellen gemessen wurde.

Nachfolgend werden zuerst anhand von Herstellungs- und Anwendungsbeispielen unter Bezugnahme auf eine Figur Vorarbeiten, Materialien und verwandte Bestimmungsverfahren beschrieben, die die schließlich die Schaffung eines erfindungsgemäßen Verfahrens möglich machten.

**Materialien und Methoden**

**Materialien**

$^{125}$I-bTSH (56μCi/μg) sowie die für die Bestimmung verwendenten kommerziellen TRAK-Assay®-Kits wurden genau wie die verwendete Protein A-Suspension und die monoclonalen Maus-Antikörper gegen die extrazelluläre Domäne des hTSHR von der Firma B.R.A.H.M.S Diagnostica GmbH, Berlin zur Verfügung gestellt. Das Plasmid pcDNA3-rLHR(B9) wurde von Dr. D.L. Segaloff (The University of Iowa, USA) zur Verfügung gestellt. Das Plasmid pSP-luc+NF wurde von der Fa. Promega (Heidelberg, Deutschland) erworben. Als ECL Western-Blot-Nachweis-Kit und cAMP-RIA-Kit wurden Kits der Fa. Amersham (Braunschweig, Deutschland) verwendet. Die DNA-Primer

```
P1 (5'-GTCATGCATCAGCTGCTGGTGCTGGCAGTG-3'),

P2 (5'-GTCGACGTCGTTATGTGTAAGTTATCACAG-3'),

P3 (5'-GTCCTTAAGAAAACACTGCCCTCCAAAGAAAAA-3')
```

und

```
P4 (5'-ATCGAGCTCTTCATTCTCCTCAAAGATGGC-3')
```

wurden von der Firma Interactiva (Ulm, Deutschland) bezogen.

**Zellkultur**

HeLa-Zellen wurden in Dulbecco's modifiziertem Eagle's Medium, das mit 10 % fetalem Rinderserum ergänzt war, gezüchtet. Die Zellen wurden in einer 5%igen $CO_2$-Atmosphäre bei 37°C kultiviert.

**Konstruktion von TSHR/LH-CGR-Chimären**

Drei unterschiedliche Chimären des humanen TSHR, bei dem Teilsequenzen durch entsprechende Sequenzen eines Ratten-LH-CGR ersetzt waren, wurden gemäß der Beschreibung in Biochem Biophys Res Commun. (1991), 179: 70-77 konstruiert. Bei einer der Chimären, die nachfolgend als "Chimäre A" bezeichnet wird, waren die TSHR-Aminosäuren 8-165 durch die vergleichbaren Aminosäuren 10-166 des LH-CGR ersetzt; bei "Chimäre B" waren die TSHR-Aminosäuren 261-370 durch die vergleichbaren Aminosäuren 261-329 eines Ratten LH-CGR ersetzt; während im Falle der "Chimäre C" sowohl die Aminosäuren 8-165 als auch die Aminosäuren 261-370 des TSHR durch vergleichbare LH-CGR-Aminosäuren ersetzt waren.

Im einzelnen wurde dabei von dem Plasmid pcDNA3-rLHR(B9) ausgegangen, das die Sequenz für den Ratten-LH-CGR-Rezeptor enthält. Die DNA-Sequenzen, die für die Aminosäuren 10-165 sowie 261-329 kodierten, wurden nach der PCR-Technik unter Verwendung der Primerpaare P1-P2 bzw. P3-P4, die NsiI, AatII, BfrI bzw. SacI-Restriktionsstellen enthielten, vervielfältigt, wodurch man NsiI/AatII bzw. BfrI/SacI-PCR-Fragmente erhielt. Parallel dazu wurde ein pTM1-TSHR-FLAG-6HIS-Plasmid, das gemäß DE 196 45 729 bzw. Exp Clin Endocrinol Diabetes. 5: 282-290 (1997) erhalten worden war, mit PstI-AatII bzw. BfrI-SacI-Restriktionsendonukleasen verdaut. Die dabei erzeugten Fragmente wurden entfernt und durch die aus dem Ratten-LH-CG-Rezeptor erhaltenen PCR-Fragmenten ersetzt, wodurch die cDNA-Sequenzen für die unterschiedlichen TSHR/LH-CGR-Chimären A, B und C erhalten wurden.

Der Vektor pTM1-TSHR-FLAG-6HIS oder der daraus erhaltene neue Vektor mit der TSHR/LH-CGR-DNA (pTM1-TSHR/LH-CGR) wurden mit ClaI linearisiert, und die "klebrigen" Enden wurden unter Verwendung von Klenow-Polymerase aufgefüllt. Das für die Expression bestimmte Fragment wurde unter Verwendung von BamHI herausgeschnitten und in die HindII/BamHI-Stelle eines Vaccinia-Transfervektors p7,5K131 subkloniert, wie in DE 196 45 729 bzw. Exp Clin Endocrinol Diabetes. 5: 282-290 (1997) näher beschrieben ist.

**Herstellung von Vektoren für die Expression von TSHR bzw. TSHR/LH-CGR-Chimären und dem Enzym Luciferase**

Unter Verwendung des Plasmids pSP-luc+NF, das die Sequenz der Leuchtkäfer-Luciferase enthält, wurden DNA-Sequenzen, die für den vollständigen humanen "Wildtyp" TSHR bzw. für TSHR/LH-CGR-Chimären kodieren, mit der cDNA verbunden, die für die 550 Aminosäuren umfassende Sequenz der Leuchtkäfer-Luciferase kodieren. Dazu wurde die Sequenz für die Leuchtkäfer-Luciferase mit BstEII und EcoRI aus dem Plasmid pSP-luc+NF geschnitten und in entsprechende Orte der p7,5K131-TSHR- bzw. p7,5K131-TSHR/LH-CGR-Vektoren eingefügt, wodurch Vaccinia-Rekombinationsplasmide p7,5K131-TSHR-LUC bzw. p7,5K131-TSHR/LH-CGR-LUC erhalten wurden.

**Erzeugung von Vacciniavirus-Rekombinanten**

Unter Anwendung der in näheren Einzelheiten in DE 196 45 729 bzw. Exp Clin Endocrinol Diabetes. 5: 282-290 (1997) oder in J Virol. 68: 8423-8427 (1994) beschriebenen Technik wurde die cDNA für das TSHR-Luciferase bzw. TSHR/LH-CGR-Luciferase-Konstrukt aus p7,5K131-TSHR-LUC bzw. p7,5K131-TSHR/LH-CGR-LUC-Plasmiden in das Genom des Wildtyp-Vacciniavirus-Stamms Kopenhagen unter homologer Rekombination in das Thymidinkinasegen inkorporiert.

**Expression und Gewinnung von Fusionsproteinen TSHR-LUC bzw. TSHR/LH-CGR-LUC als Zellextrakt**

Konfluente HeLa-Zellen, die in einer 75 cm$^2$-Platte (ungefähr 20 x 10$^6$ Zellen) gezüchtet wurden, wurden mit einer koloniebildenden Einheit (cfu) an rekombinantem Vacciniavirus pro Zelle infiziert und für 24 h bei 37°C inkubiert. Infizierte Zellen wurden durch Abschaben in eine phosphatgepufferet Salzlösung (PBS) überführt und viermal mit PBS unter Zentrifugieren bei 2500 U/min gewaschen. Die erhaltenen Zellen wurden in 0,3 ml eines Puffers A (20mM Hepes-KOH; pH 7,5; 50 mM NaCl; 1 % Triton X100; 10 % Glycerol) unter Einfrieren und Auftauen lysiert. Die erhaltene Suspension wurde bei 30000 g 1h zentrifugiert, der Überstand (etwa 8 mg/ml Gesamtprotein) wurde gesammelt und bei -70°C aufbewahrt.

Der wie beschrieben erhaltene Überstand (Extrakt) wurde in den Immunpräzipitations-Versuchen als TSHR-LUC bzw. TSHR/LH-CGR-LUC eingesetzt.

**Herstellung von Zellfraktionen**

Wie oben beschrieben wurde eine Monoschicht von konfluenten HeLa-Zellen, die in einer 75 cm$^2$-Platte gezüchtet wurden, mit einer Plaque-bildenden Einheit eines rekombinanten Vacciniavirus pro Zelle infiziert und 24 h bei 37°C inkubiert. Infizierte Zellen wurden mit PBS gewaschen, geerntet und unter Zentrifugieren bei 1200 U/min pelletiert. Das erhaltene Zellpellet wurde in 0,3 ml eines Puffers resuspendiert, der 10 mM Tris-HCl, pH 7,6, 50 mM NaCl, 10% Glycerol sowie eine Proteaseinhibitorenmischung enthielt. Die Suspension wurde danach bei 4°C durch 20 Hubbewegungen in einem Glas/Teflon-Homogenisator homogenisiert und dann 15 min bei 800 g zentrifugiert sowie anschließend für 1 h bei 30000 g. Der Überstand (die Cytoplasmafraktion) wurde gesammelt. Das Membranpellet wurde aufgearbeitet, indem es bei 4°C durch 20 Hubbewegungen in einem Glas/Teflon-Homogenisator in 0,3 ml 1% Triton X100 im gleichen Puffer homogenisiert und dann bei 30000 g für 1 h zentrifugiert wurde. Der Überstand (Triton X100-Membranextrakt) wurde gesammelt und bei -70°C aufbewahrt.

## Immunpräzipitations-Versuche

Für die nachfolgend beschriebenen Immunpräzipitations-Analysen wurden 40 µl TSHR-LUC bzw. TSHR/LH-CGR-LUC ($2x10^6$ relative Lichteinheiten) mit 10 µl Serum vermischt und über Nacht bei 4°C inkubiert. Zur Ausfällung der gebildeten Immunkomplexe wurden 25 µl einer 20%igen Protein A-Suspension in Puffer A zugesetzt, und die Mischung wurde unter Schütteln 1 h bei 4°C inkubiert. Unter Zentrifugieren wurde die Suspension 4 mal mit 1 ml Puffer A (ohne Triton X100) gewaschen. Abschließend wurde das Pellet in 50 µl Puffer A (ohne Triton X100) resuspendiert, und die Luciferaseaktivität in der aus dem Pellet erhaltenen Suspension wurde mittels eines Luminometers (Lumat 9501, Firma Berthold, Wildbad, Germany) nach dem üblichen Bestimmungsprotokoll bestimmt. Die erhaltenen Ergebnisse wurden als gebundene relative Lichteinheiten ausgedrückt.

## Bindung von $^{125}$I-TSH an TSHR-LUC oder TSHR/LH-CGR-LUC

Die Prüfung der Bindung von $^{125}$I-TSH an die verschiedenen solubilisierten Luciferase-Fusionsproteine wurde in einem Gesamtvolumen von 200 µl durchgeführt, das sich zusammensetzte aus 50 µl Fusionsprotein-Lösung (Zellextrakt), 50 µl Nullstandard-Serum sowie 100 µl einer $^{125}$I-TSH-Lösung (etwa 20000 cpm). Die Reaktionsmischung wurde 2 h bei Raumtemperatur inkubiert, wonach die Komplexe aus $^{125}$I-TSH/Fusionsproteinen durch Zugabe von 2 ml Polyethylenglycol ausgefällt wurden. Nach Zentrifugieren bei 2000 g für 10 min wurde die Gammastrahlung im Pellet gemessen.

Das Ausmaß der unspezifischen Bindung wurde in Gegenwart von $10^{-7}$ M TSH bestimmt und betrug < 5% der Gesamtbindung.

## Bestimmung von die TSH-Bindung inhibierenden Immunglobulinen (TBII)

Die Bestimmung der TSHR-Auto-Ab von Typ der die TSH-Bindung inhibierenden Immunglobuline (TBII) wurde unter Verwendung von Teströhrchen, die an ihren Wänden einen über Antikörper affinitäts-immobilisierten rhTSHR aufweisen (TRAK Assay® der Fa. B.R.A.H.M.S Diagnostica GmbH; vgl. auch J Clin Endocrinol Metab. 84:90-97, 1999) , gemäß den Vorschriften des Herstellers durchgeführt. Die Ergebnisse wurden, unter Verwendung einer Standardkurve (Verdrängungskurve mit Standardseren 0, 1, 2, 4, 16, 40 IU/l aus dem TRAK Assay®), in internationalen Einheiten/Liter (IU/l) angegeben. Die Menge an Autoantikörpern im Testserum ist proportional zu dem gemessenen Wert (IU/l).

## Bioassay zum Nachweis von stimulierenden TSHR-Auto-Ab (TSAb)

Der Nachweis von TSAb wurde in unfraktionierten Seren unter Verwendung von CHO-Zellen durchgeführt, die den TSHR stabil exprimieren. Die Bestimmung von extrazellulärem cAMP im Überstand wurde mit einem kommerziellen cAMP-RIA-Kit entsprechend den Vorschriften des Herstellers durchgeführt. Der Stimulierungsindex (SI) wurde entsprechend der Veröffentlichung Vlase H et al. in Endocrinology (1995), 136: 4415-4423 wie folgt berechnet:

$$SI(\%) = 100 \times (cAMP \text{ Patient} / cAMP \text{ negative Kontrollprobe}).$$

## Bioassay zum Nachweis von blockierenden TSHR-Auto-Ab (TBAb)

Bovines TSH (10 mU/ml) wurde zu CHO-Zellen, die den hTSHR stabil exprimieren, entweder mit Kontroll- oder Testserum zugefügt. Die jeweilige cAMP-Produktion wurde, wie kürzlich beschrieben (Wallaschofski und Paschke, Clin Endocrinol. 50:365-372, 1999) gemessen und verglichen. Die Bestimmung von extrazellulärem cAMP im Überstand wurde mit einem kommerziellen cAMP-RIA-Kit entsprechend den Vorschriften des Herstellers durchgeführt. Der Inhibierungsindex (II) wurde berechnet als:

$$II(\%) = 100 \times [1-(cAMP \text{ Patient TSH} / cAMP \text{ negative Kontrolle}$$

$$TSH)].$$

**Western blotting**

Proteine wurden einer Polyacrylgelelektrophorese (PAGE) in 8% SDS unterworfen und auf übliche Weise auf Nitrozellulosemembranen übertragen. Die Membranen wurden über Nacht bei 4°C mit monoklonalen Mäuse-Antikörpern gegen die Aminosäuren 378-388 der extrazellulären Domäne des TSHR in einer Verdünnung von 1:20 versetzt. Gebundene Antikörper wurden anschließend unter Verwendung eines Western blotting Kits für eine verstärkte Chemilumineszenz nachgewiesen.

Unter Verwendung der obigen Materialien und unter Anwendung der beschriebenen Techniken wurden die nachfolgend beschriebenen Versuche durchgeführt, deren Ergebnisse unter Bezugnahme auf die Figuren näher erläutert werden.

In den Figuren zeigen:

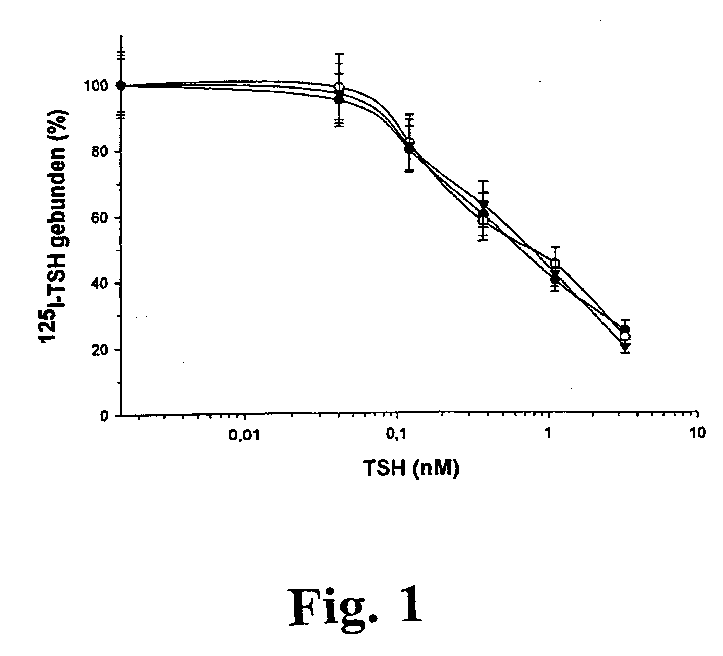

Fig. 1   die Bindung von TSH an verschiedene exprimierte rhTSHR-Produkte, und zwar in Form von Verdrängungskurven, die die Inhibierung der Bindung von $^{125}$I-bTSH durch steigende Mengen an unmarkiertem TSH an Wildtyp-TSHR (WT; ■); TSHR-LUC (o) sowie an Chimäre A-LUC (▲) zeigen. Die Ergebnisse sind ausgedrückt als Prozent der Gesamtbindung in Abwesenheit von unmarkiertem TSH, die für WTTSHR, TSHR-LUC und Chimäre A-LUC 4512 cpm, 2793 cpm bzw. 2856 cpm betrugen. Jeder Wert ist der Mittelwert ± 2 SD von Doppelmessungen in zwei getrennten Versuchen.

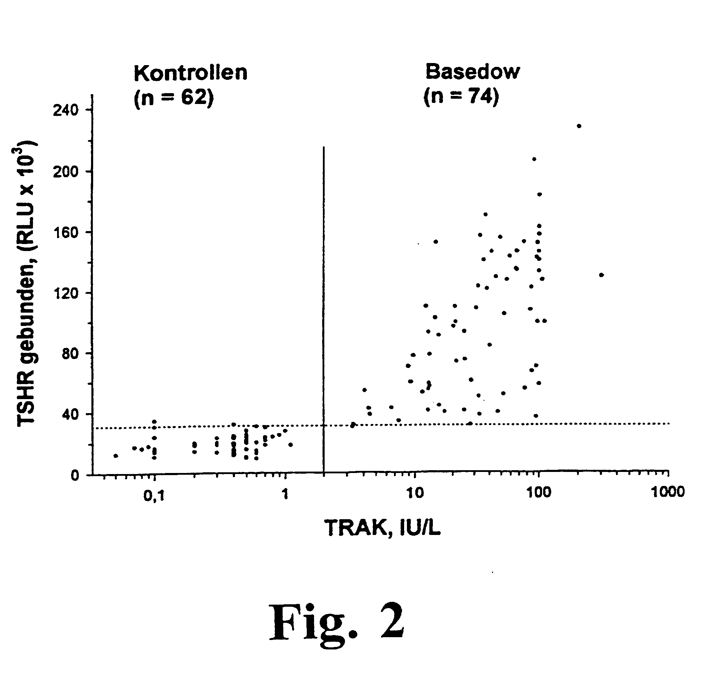

Fig. 2   die Fähigkeit von Kontrollseren und Seren von Morbus Basedow-Patienten zur Immunpräzipitation von WTTSHR-LUC im Vergleich mit der Fähigkeit dieser Seren, in einem herkömmlichen kompetitiven TRAK Assay die Bindung von $^{125}$I-bTSH an ein hrTSHR-Präparat zu inhibieren. Die Immunpräzipitations-Aktivitäten der Seren sind ausgedrückt als gebundene RLU (relative Lichteinheiten), die Inhibierungsaktivitäten werden als internationale Einheiten/Liter (IU/l) ausgedrückt, wie im Text näher erläutert wird. Die waagerechte punktierte Linie ist die Linie für zwei Standardabweichungen (SD) oberhalb des Mittelwerts für Normalpersonen (30737 RLU).

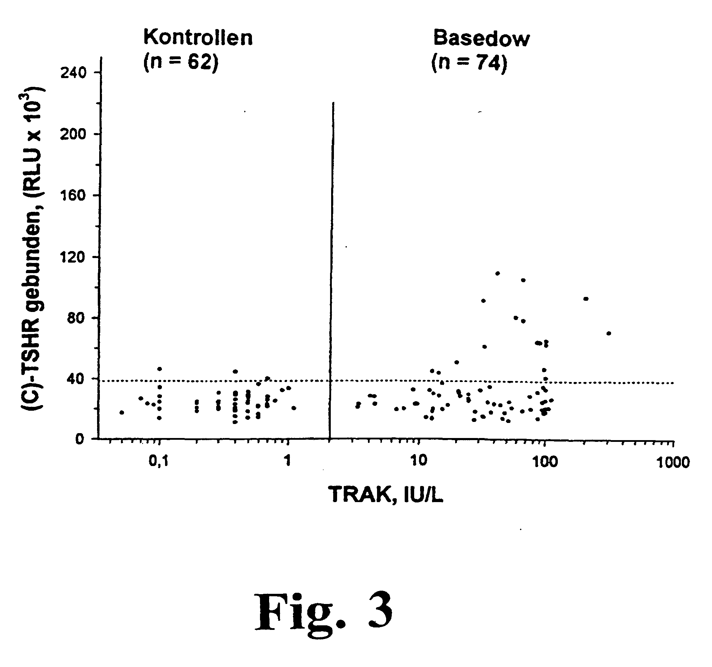

Fig. 3   die Fähigkeit von Kontrollseren und Seren von Morbus Basedow-Patienten zur Immunpräzipitation von Chimäre C-LUC im Vergleich mit der Fähigkeit dieser Seren, in einem herkömmlichen kompetitiven TRAK Assay® die Bindung von $^{125}$I-bTSH an ein hrTSHR-Präparat zu inhibieren. Die Immunpräzipitations-Aktivitäten der Seren sind ausgedrückt als gebundene RLU (relative Lichteinheiten), die Inhibierungsaktivitäten werden als internationale Einheiten/pro Liter (IU/l) ausgedrückt, wie im Text näher erläutert wird. Die waagerechte punktierte Linie ist die Linie für zwei Standardabweichungen (SD) oberhalb des Mittelwerts für Normalpersonen (38980 RLU).

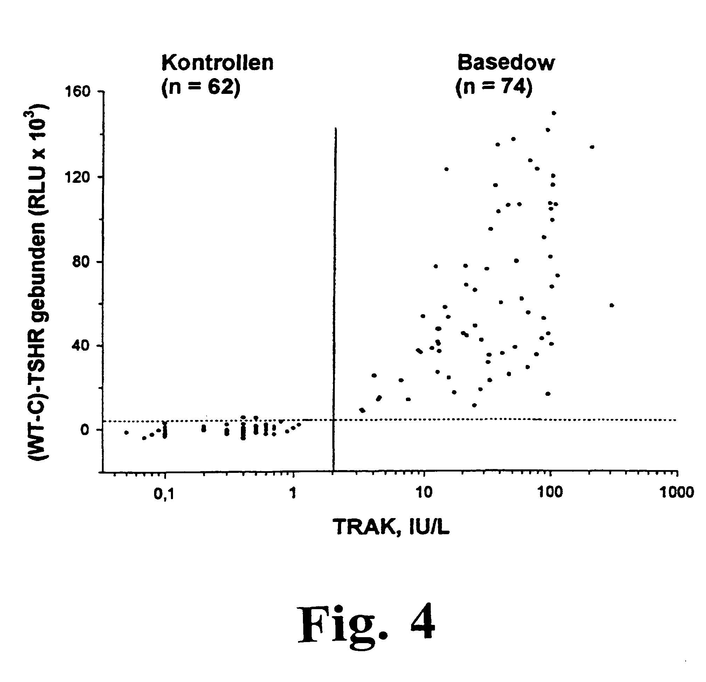

Fig. 4   die Differenz der Immunpräzipitatbildung von TSHR-LUC und Chimäre C-LUC (WT-C) mit Seren von Normalpersonen und Morbus Basedow-Patienten, verglichen mit der Fähigkeit dieser Seren, die Bindung von $^{125}$I-TSH an ein rhTSHR-Präparat zu inhibieren. Die Immunpräzipitations-Aktivitäten der Seren sind als Werte von gebundenem RLU ausgedrückt, die Inhibierungsaktivitäten werden, wie im Text erläutert, in IU/l ausgedrückt. Die waagerechte punktierte Linie gibt den Wert von zwei Standardabweichungen (SD) oberhalb des Mittelwerts für Normalpatienten (3384 RLU) an.

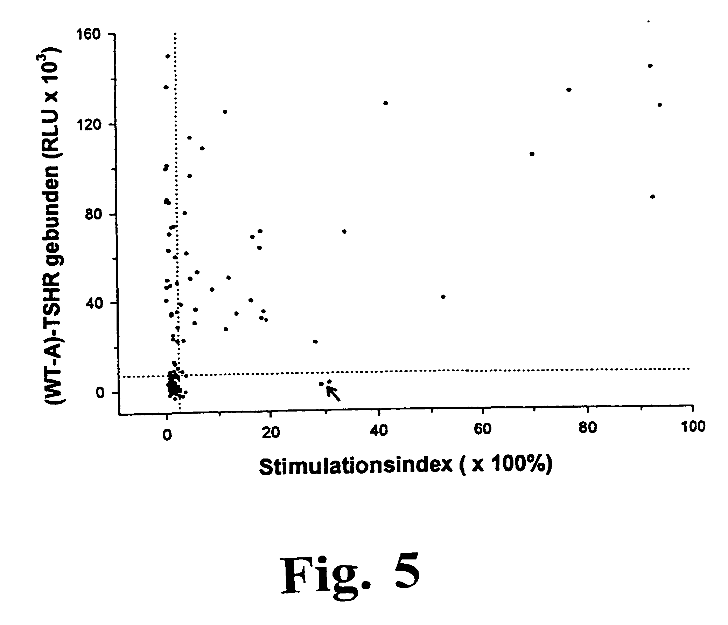

Fig. 5   die Differenz der Immunpräzipitatbildung von TSHR-LUC und Chimäre A-LUC (WT-A) in Seren von Normalpatienten (n=62) und Seren von Morbus Basedow-Patienten (n=63) verglichen mit der Fähigkeit dieser Seren, in einem cAMP-Bioassay die cAMP-Synthese zu stimulieren. Die Immunpräzipitations-Aktivitäten der Seren sind ausgedrückt als gebundene RLU, die cAMP-stimulierenden Aktivitäten werden als Stimulationsindex (SI) ausgedrückt. Die waagerechte punktierte Linie ist die Linie für zwei Standardabweichungen (SD) oberhalb des Mittelwerts für normale Patienten (7000 RLU). Die senkrechte gepunktete Linie zeigt den SI-cutoff für Normalseren (200%).

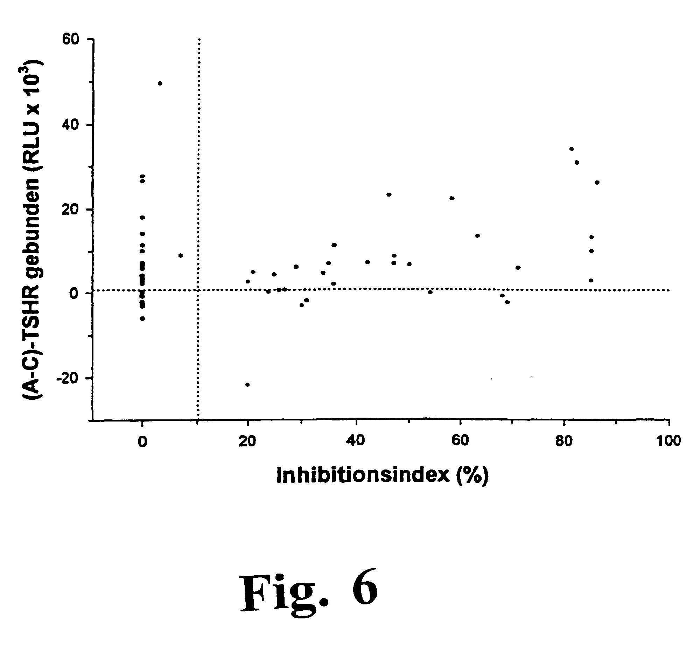

Fig. 6   die Differenz der Immunpräzipitatbildung der Chimären A und C (A-C) durch Seren von Morbus Basedow-Patienten (n=63) verglichen mit der Fähigkeit dieser Seren, die cAMP-Synthese in einem cAMP-Bioassay zu inhibieren. Die Immunpräzipitations-Aktivitäten der Seren sind ausgedrückt als gebundene RLU, die cAMP-blockierenden Aktivitäten sind ausgedrückt als Inhibierungsindex (II). Die waagerechte gepunktete Linie zeigt den Wert von zwei Standardabweichungen (SD) oberhalb des Mittelwerts für Normalpersonen (835 RLU). Die senkrechte punktierte Linie zeigt den II-cutoff für Seren von Normalpersonen (10%).

Nachfolgend werden die verschiedenen Messungen unter Verwendung der neuen TSHR-LUC bzw. TSHR/LH-CGR-LUC Fusionsprodukte näher beschrieben.

**Reaktivität von TSHR-LUC bzw. TSHR/LH-CGR-LUC-Fusionsprodukten mit $^{125}$I-TSH**

Zur Prüfung der Frage, ob die Luciferase-Fusionsprodukte gegenüber TSH ein Bindungsverhalten zeigen, das den Erwartungen entspricht, wurden die die entsprechenden Fusionsprodukte enthaltenden Lysate von HeLa-Zellen, die mit einem geeigneten rekombinanten Vacciniavirus gemäß den obigen Versuchen infiziert worden waren, mit $^{125}$I-TSH umgesetzt, und es wurde der Grad der Bindung von $^{125}$I-TSH in Abhängigkeit von der gleichzeitig in der Reaktionslösung vorhandenen Menge an unmarkiertem TSH gemessen. Die Ergebnisse der Versuche sind in Fig. 1 dargestellt, in der der Wert für 100% die Gesamtmenge an gebundenem $^{125}$I-TSH in Abwesenheit von unmarkiertem TSH darstellt. Es zeigte sich, daß keine grundsätzlichen Unterschiede bei den Verdrängungskurven zu erkennen sind, die man für die Fusionsproteine TSHR-LUC und die Chimäre A-LUC, bei der die TSHR-Aminosäuren 8-165 ersetzt sind, erhielt. Der Absolutwert für die maximale TSH-Bindung war für den Wildtyp TSHR etwa 1,6 mal höher als für die LUC-Fusionsprodukte. Berücksichtigt man, daß bei der Expression von Wildtyp-TSHR in HeLa-Zellen eine Rezeptordichte von etwa 150000 Rezeptoren pro Zelle erhalten wird, läßt sich errechnen, daß derartige HeLa-Zellen etwa zwei Drittel dieser Menge, d.h. etwa 100000 Rezeptor-Luciferase-Fusionsmoleküle pro Zelle erzeugten.

Die anderen Chimären-Fusionsprodukten, nämlich Chimäre B-LUC und Chimäre C-LUC, bei denen die TSHR-Reste 261-370 bzw. die TSHR-Reste 8-165 sowie 261-370 durch Ratten-LH-CGR-Reste ersetzt waren, waren nicht in der Lage, $^{125}$I-TSH spezifisch zu binden. Diese Ergebnisse sind in Übereinstimmung mit dem, was aufgrund von Literaturdaten erwartet werden konnte (Biochem Biophys Res Commun. 179: 70-77 (1991), J Clin Endocrinol Metab. 81: 1758-1767 (1996)).

Es ist ausdrücklich darauf hinzuweisen, daß alle Fusionsprodukte die enzymatische Aktivität der Leuchtkäfer-Luciferase aufwiesen. Die Lumineszenzintensität von Zelllysaten, die die Fusionsprodukte TSHR-LUC bzw. TSHR/LH-CGR-LUC enthielten, war weitgehend identisch, indem pro ml Lysat etwa $10^9$ RLU gemessen wurden.

Wie die oben beschriebenen Versuche zur Herstellung von Zellmembranfraktionen ergaben, war in allen Fällen mehr als 90% der Luciferaseaktivität in der Membranfraktion lokalisiert. Nur etwa 10% der Luciferaseaktivität wurde in der wasserlöslichen Cytoplasmafraktion gefunden. Die Luciferase-Meßdaten zeigten genau wie Western-Blotting-Experimente, daß alle Fusionsprodukte in HeLa-Zellen nach der beschriebenen Technik mit etwa der gleichen Wirksamkeit exprimiert wurden.

**Nachweis von TSHR-Auto-Ab in Seren von Morbus Basedow-Patienten unter Verwendung der TSHR-LUC-Fusionsprodukte sowie unter Verwendung der verschiedenen TSHR/LH-CGR-LUC-Fusionsprodukte.**

TSHR-LUC und TSHR/LH-CGR-LUC-Fusionsprodukte wurden zum Nachweis von Autoantikörpern in Seren von Morbus Basedow-Patienten durch Immunpräzipitationsanalyse nach der weiter vorn beschriebenen allgemeinen Arbeitsweise eingesetzt. Wenn man davon ausgeht, daß die Epitope für TSAb im Bereich der Aminosäuren 25-165 des TSHR lokalisiert sind, und die Epitope für TBAb im Bereich der Aminosäuren 261-370, lassen sich die Meßergebnisse der Immunpräzipitationsanalyse zur Bestimmung der Relativmengen der unterschiedlichen Typen von Autoantikörpern in Seren von Morbus Basedow-Patienten heranziehen. Es sollte folgendes gelten:

(i) die Bindung von Fusionsprodukten, die WT TSHR (WT) enthalten, sollte der Gesamtmenge an TSHR-Auto-Ab in den Seren proportional sein,
(ii) der Unterschied der Werte für die Bindung von TSHR-LUC und Chimäre A-LUC (WT-A) (oder zwischen den Chimären B-LUC und C-LUC, d.h. B-C) durch die Testseren sollte der Menge von TSAb in diesen Seren entsprechen,
(iii) der Unterschied zwischen den Bindungswerten für TSHR-LUC und Chimäre B-LUC (WT-B) (oder zwischen den Chimären A-LUC und C-LUC, d.h. A-C) sollte die Menge an TBAb in Seren widerspiegeln, (iv) das Ausmaß der Bindung an Chimäre C-LUC, die weder die Epitope für TSAb noch für TBAb enthält, sollte die Menge an neutralen Autoantikörpern in den Testseren widerspiegeln.

Es zeigte sich, daß die Fusionsprodukte von sowohl TSHR als auch den drei TSHR/LH-CGR-Chimären eine deutliche Wechselwirkung mit den in Seren von Morbus Basedow-Patienten vorhandenen Autoantikörpern zeigten. Tabelle 1 zeigt die mittleren Bindungswerte, die unter Verwendung von Seren von 62 Kontrollpersonen und 74 Morbus Basedow-Patienten erhalten wurden.

Tabelle 1

|  | TSHR | Chimäre A | Chimäre B | Chimäre C |
|---|---|---|---|---|
| Kontrollen (n=62) | 19573 | 17965 | 22251 | 22794 |
| Patienten (n=74) | 97065 | 40209 | 130481 | 33832 |

Die Fusionsprodukte der Chimären A und C (die keine Epitope für TSAb aufweisen) wechselwirkten mit pathologischen Autoantikörpern in allen 74 Fällen schlechter als WT-TSHR-Produkte. Unerwarteterweise zeigte sich, daß das Fusionsprodukt der Chimäre B (die keine Epitope für TBAb enthält) die pathologischen Autoantikörper in allen Fällen wirksamer band als Fusionsprodukte des WT-TSHR. Möglicherweise ist das auf eine für die TSAb-Bindung vorteilhafte Veränderung der Rezeptorstruktur zurückzuführen.

Ferner zeigte sich, daß Seren von Normalpersonen eine Bindung an alle Fusionsprodukte, d.h. WT-TSHR und alle Chimären, mit etwa der gleichen Wirksamkeit zeigten, wobei die Unterschiede in allen Fällen im Bereich von 10 bis 15% lagen. Die in Tabelle 1 gezeigten Ergebnisse gestatten es, zu errechnen, daß der mittlere Gehalt der verschiedenen Autoantikörpertypen für die 74 vermessenen Seren von Morbus Basedow-Patienten etwa 59% TSAb, etwa 7% TBAb und etwa 35% neutrale TSHR-Auto-Ab, angegeben als Prozentsatz bezogen auf die Gesamtmenge an TSHR-Auto-Ab, betrug. Es bestand eine positive Korrelation der Bindungswerte für jede Kombination von Fusionsprodukten, wenn ihr Immunpräzipitationsverhalten in 74 Seren von Morbus Basedow-Patienten verglichen wurde (r im Bereich von 0,57 bis 0,91; p < 0,001, vgl. Tabelle 2).

Tabelle 2

| Verglichene Chimären | WT und A | WT und B | WT und C | A und B | A und C | B und C |
|---|---|---|---|---|---|---|
| Korrelation | 0,60 | 0,89 | 0,57 | 0,63 | 0,91 | 0,66 |

Die Gesamtmenge an TSHR-Auto-Ab in den Testseren wurde unter Verwendung des mit Luciferase fusionierten Wildtyp-TSHR bestimmt. Die Spezifität bei einer TSHR-LUC-Immunpräzipitation unter Verwendung von 62 Kontrollseren und 74 Seren von Patienten mit Morbus Basedow verschiedener Schwere ist in Fig. 2 gezeigt. Nur zwei der 62 Normalseren zeigten mit dem TSHRhaltigen Fusionsprodukt eine Immunpräzipitationsaktivität, die höher war als ein Wert, der dem Mittelwert für Normalpersonen zuzüglicher zweier Standardabweichungen entsprach (30740 RLU). Die Bindungswerte für alle anderen Kontrollseren lagen im Bereich von 10000 bis 30000 RLU. Das separat bestimmte Niveau der unspezifischen Bindung von TSHR-LUC durch die Protein A-Suspension in Abwesenheit eines Serums betrug nur etwa 1.000 RLU. Im Gegensatz zu den Kontrollseren wurden 73 von 74 Seren von Morbus Basedow-Patienten in dem Immunpräzipitationsassay als positiv gemessen. In diesen Fällen lag der Bindungsgrad im Bereich von 30000 bis 240000 RLU.

Die gezeigten Daten deuten auf eine hohe Spezifität des TSHR-LUC bzw. TSHR/LH-CGR-LUC-Immunpräzipitationsassays. Bei einem direkten Vergleich wurde auch eine positive Korrelation (r = 0,61; p < 0,001) zwischen den mit dem Immunpräzipitationsassay erhaltenen Ergebnissen und den Ergebnissen einer TBII-Messung der 74 Seren von Morbus Basedow-Patienten mit dem kompetitiven TRAK Assay® erhalten. Kontrollversuche ergaben, daß Seren von Patienten, die an einer anderen Autoimmunerkrankung litten (Seren von zehn Patienten mit juvenilem Diabetes mellitus) TSHR-Fusionsprodukte nur mit einer sehr niedrigen Wirksamkeit präzipitierten. Die Präzipitationsaktivitäten waren dabei vergleichbar mit denen, die für Normalpersonen erhalten wurden, indem die Bindungswerte im Bereich von 10000 bis 23000 RLU lagen.

**Nachweis von "neutralen" TSHR-Auto-Ab in Seren von Morbus Basedow-Patienten unter Verwendung von TSHR/LH-CGR-LUC-Chimären**

Die Menge an "neutralen TSHR-Auto-Ab" in den Testseren wurde unter Verwendung des Fusionsprodukts Chimäre C-LUC ermittelt. Es zeigte sich, daß derartige neutrale TSHR-Auto-Ab in allen 62 Seren der Kontrollpersonen sowie in allen 74 Seren von Morbus Basedow-Patienten nachzuweisen waren. Die Bindungswerte für die Kontrollseren lagen im Bereich von 10000 bis 50000 RLU, während sie für die Patientenseren im Bereich von 10000 bis 110000 RLU lagen. Die meisten der Patientenseren (57 von 74) enthalten etwa die gleiche Menge an neutralen TSHR-Auto-Ab wie die Kontrollseren. Die Relativmenge der neutralen TSHR-Auto-Ab in den Patientenseren liegt im Bereich von 10 bis 75% der Gesamtmenge an TSHR-Auto-Ab, wohingegen praktisch alle TSHR-Auto-Ab in den Kontrollseren zu den neutralen TSHR-Auto-Ab gehören (90-100%). Fig. 4 zeigt die Unterschiede des Präzipitationsgrads mit Fusionsprodukten des Wildtyp-TSHR und der Chimäre C bei den gleichen Seren. Die erhaltenen Werte ergeben die Summe aus TSAb und TBAb (ohne neutrale Antikörper) in den untersuchten Seren. In diesem Fall ist der Unterschied zwischen Kontrollseren und Patientenseren stärker ausgeprägt, indem alle 74 Patientenseren im vorliegenden Assay klar positiv waren. Von den Kontrollseren andererseits waren nur zwei als positiv anzusehen, was darauf hinweist, daß möglicherweise einige Normalpersonen eine gewisse Menge an sowohl stimulierenden als auch blockierenden TSHR-Auto-Ab enthalten können. Die Korrelation zwischen den bei diesem Assay und dem TRAK Assay® erhaltenen Ergebnissen war positiv (r = 0,56; p < 0,001; n = 74).

**Nachweis von TSAb in Seren von Morbus Basedow-Patienten unter Verwendung von TSHR/LH-CGR-LUC-Chimären**

Die Menge an TSAb (stimulierenden TSHR-Auto-Ab) in Patientenseren wurde aufgrund des Unterschieds der Bindungswirksamkeit der Fusionsprodukte mit dem WT-TSHR bzw. derjenigen mit der Chimäre A errechnet. Fig. 5 zeigt den Vergleich für den Stimulationsindex (SI) von 62 Kontrollseren und 63 Patientenseren, bestimmt in einem herkömmlichen cAMP-Bioassay, gegenübergestellt dem Unterschied der Bindungswirksamkeit der Fusionsprodukte mit WT-TSHR bzw. Chimäre A (WT-A) in diesen Seren. Im Immunpräzipitationsassay waren 59 von 62 Kontrollseren negativ (Bindungswirksamkeit < 7000 RLU), während drei Seren als schwach positiv angesehen werden können (Bindungswirksamkeiten von 8400, 7340 bzw. 7160 RLU). Zwei der Normalseren, auf die in Fig. 5 mit einem Pfeil verwiesen wird, waren im cAMP-Bioassay positiv (SI = 2930 bzw. 3080%; SI-cutoff für Normalpersonen liegt im Bereich von 200%). Diese beiden Patienten waren jedoch nach Maßgabe des Immunpräzipitationsassays sowie der klinischen Bewertung keine Morbus Basedow-Patienten. Bei den Seren von Morbus Basedow-Patienten waren 40 von 63 Seren (63%) im cAMP-Bioassay positiv (SI $\geq$ 200%), während im Immunpräzipitationsassay 61 von 63 Seren (97%) positiv waren (WT-A) . Es bestand eine positive Korrelation zwischen den Ergebnissen, die im cAMP-Bioassay erhalten wurden, und denen, die im Immunpräzipitationsassay (WT-A) erhalten wurden (r = 0,43; p < 0,001; n = 63). Wenn man nur solche Seren berücksichtigte, die auch im cAMP-Bioassay positiv reagierten (SI$\geq$200%), dann wurde die Korrelation sehr viel besser (r = 0,61; p < 0,001; n = 40).

**Nachweis von TBAb in Seren von Morbus Basedow-Patienten unter Verwendung von TSHR/LH-CGR-LUC-Chimären**

TBAb wurden in den Patientenseren anhand des Unterschieds des Bindungsverhaltens der LUC-Fusionsprodukte der Chimären A und C bestimmt. Fig. 6 zeigt den Vergleich des Inhibierungsindex (II) von 62 Kontrollseren und 63 Patientenseren (bestimmt im cAMP-Bioassay) mit dem Unterschied des Bindungsverhaltens für die Fusionsprodukte mit den Chimären A und C (A-C). 58 von 62 Kontrollseren waren im cAMP-Bioassay negativ (II < 10%), und alle Kontrollseren waren im Immunpräzipitationsassay negativ (Bindungswirksamkeit < 835 RLU). Unter Verwendung des cAMP-Bioassays wurde in 31 von 63 Patientenseren (49%) eine TBAb-Aktivität festgestellt, während im Immunpräzipitationsassay (A-C) 45 von 63 Patientenseren (71%) als TBAb-positiv ermittelt wurden. Neun Immunopräzipitationspositive Basedow-Seren waren negativ im cAMP-Bioassay, und umgekehrt waren neun cAMP-Bioassay-positive Seren im Immunopräzipitationsassay negativ. Es bestand eine schwache positive Korrelation zwischen den Ergebnissen im cAMP-Bioassay und denen vom (A-C)-Immunopräzipitationsassay (r=0,25; p < 0,05; n = 63). Wenn man nur solche Patientenseren berücksichtigte, die im cAMP-Bioassay als TBAb-positiv gemessen wurden (II $\geq$ 10%), wurde jedoch eine signifikante Korrelation erhalten (r = 0,57; p < 0,001; n = 31). Eine Korrelation zwischen den II-Werten von unterschiedlichen Patientenseren und der Fähigkeit dieser Seren, TSHR- oder TSHR/LH-CGR-Fusionsprodukte zu präzipitieren, bestand nicht (Daten sind nicht gezeigt).

Die angeführten Daten zeigen eine hohe Sensitivität und Spezifität eines Immunpräzipitationsassays unter Verwendung von enzymmarkierten Fusionsprodukten, die TSHR/LH-CGR-Chimären bzw. den Wildtyp TSHR enthielten. Damit erweist sich der Assay als vielversprechendes Instrument zur Diagnose und Untersuchung des Ausbruchs, der Remission und des Wiederauftretens von Morbus Basedow. Nachdem die bisherigen Versuche unter Verwendung von Fusionsprodukten mit Leuchtkäfer-Luciferase als Enzymrest erfolgreich verliefen, kann davon ausgegangen werden, daß entsprechende Assays auch mit anderen Enzymresten, die sich insbesondere unter Kostenaspekten besser für eine praktische klinische Anwendung eignen und die Komponenten bekannter Enzymassay-Nachweissysteme sind, herstellen lassen. Insbesondere ist dabei an Enzymreste zu denken, die ausgewählt sind aus Meerrettich-Peroxidase, alkalische Phosphatase, β-Galactosidase oder Chloramphenicol-Acetyltransferase.

Die obigen Ergebnisse zeigen anhand von verschiedenen Meßreihen, die jeweils mit einzelnen TSHR- bzw. TSHR-Chimären-Fusionsprodukten, d.h. für eine Chemiluminiszenz-Nachweisreaktion geeignet enzymmarkierten rTSHR bzw. rTHSR-Chimären durchgeführt wurden, daß sich unter Verwendung derartiger Chimären spezifische Gruppen von TSHR-Auto-Ab, nämlich TSAb, TBAb sowie "neutrale TSHR-Auto-Ab" (nachfolgend abgekürzt als "TNAb") selektiv bestimmen lassen, ohne daß auf Bioassays (cAMP-Bestimmungen) zurückgegriffen werden muß.

Um einen bestimmten einzelnen Typ von diagnostisch relevanten TSHR-Auto-Ab, d.h. insbesondere TSAb oder TBAb, zu bestimmen, war es dabei erforderlich, eine Differenz zweier Meßergebnisse zu bilden, und zwar unter Beachtung der folgenden, die o.g. Ergebnisse zusammenfassenden schematischen Beziehungen (WT-TSHR, WT-A, WT-B und WT-C standen dabei für die mit den jeweiligen Fusionsprodukten in den Immunpräzipitationsassays erhaltenen, ggf. zu korrigierenden Bindungswerte) :

| WT-TSHR (= LUC-TSHR) | bindet | $\Sigma$ TSAb + TBAb + TNAb |
|---|---|---|
| WT-A (= Chimäre A-LUC) | bindet | $\Sigma$ TBAb + TNAb |
| WT-B (= Chimäre B-LUC) | bindet | $\Sigma$ TSAb + TNAb |
| WT-C (= Chimäre C-LUC) | bindet | TNAb. |

Daraus folgte:

$$TSAb = WT\text{-}TSHR - WT\text{-}A^* \text{ (oder (WT-A)-TSHR in Fig.5),}$$

oder

$$TSAb = WT\text{-}B - WT\text{-}C^*;$$

$$TBAb = WT\text{-}TSHR - WT\text{-}B^*, \text{ oder}$$

oder

$$TBAb = WT\text{-}A - WT\text{-}C^* \text{ (oder (A-C)-TSHR in Fig.6);}$$

$$TSAb + TBAb = WT\text{-}TSHR - WT\text{-}C^* \text{ (oder (WT-C)-TSHR in Fig.4);}$$

$$TNAb = WT\text{-}C \text{ (oder (C)-TSHR in Fig.3).}$$

Die aufgrund der o.g. Differenzmessungen erhaltenen Befunde lassen sich nunmehr dazu nutzen, für die praktische klinische Anwendung geeignetere Assays zu konzipieren, bei denen man die Chimären so einsetzt, daß eine Differenzbildung im obigen Sinne nicht rechnerisch im Sinne einer Auswertung unterschiedlicher Meßreihen erfolgt, sondern direkt in vitro, indem man den in Abzug zu bringenden Term (*) direkt bei der Erzeugung des Meßsignals ausschließt, und zwar insbesondere im Sinne einer nicht zu einem Meßsignal führenden Neutralisierungsreaktion. Dabei arbeitet man wenigstens teilweise mit den an sich bekannten, unmarkierten TSHR-Chimären A, B und C, die zur möglichst vollständigen Neutralisierung der nicht zu erfassenden TSHR-Auto-Ab in der Regel im Überschuß einzusetzen sind. Folgende derartige Arbeitsweisen sind dabei hervorzuheben:

**Selektiver einstufiger Immunpräzipitationsassay (Direktbindungsassay) für TBAb (oder TSAb)**

Wie in den o.g. Versuchen wird mit einem markierten Wildtyp-TSHR WT-TSHR, z.B. mit dem direkt markierten LUC-TSHR, gearbeitet. Die Probe wird jedoch vor der Umsetzung mit diesem WT-TSHR oder parallel dazu mit einem Überschuß der unmarkierten Chimäre B (WT-B) versetzt, die im Idealfall mit allen in der Probe enthaltenen stimulierenden und neutralen TSHR-Auto-Ab (d.h. mit allen TSAb und TNAb) reagiert und diese TSHR-Auto-Ab-Typen der Umsetzung mit dem markierten WT-TSHR entzieht.
Bei der Umsetzung werden somit nur die Komplexe aus WT-TSHR/TBAb in markierter Form erhalten, und nur diese tragen nach der Fällungsreaktion, z.B. nach Fällung aller Auto-Ab enthaltenden Immunkomplexe mit Protein A, zur Erzeugung eines Meßsignals bei, das deshalb direkt der Menge an TBAb in der Probe proportional ist. Damit ist jedoch eine selektive Messung von TBAb mit nur einer Bestimmung möglich.
Arbeitet man im o.g. Immunpräzipitationsassay mit einem Zusatz der Chimäre A, erfaßt man entsprechend nur die stimulierenden TSHR-Auto-Ab.
Es kann dabei vorteilhaft sein, die Probe vor ihrer Umsetzung mit dem markierten WT-TSHR einer Vorinkubation mit der jeweiligen Chimäre zu unterziehen, um die Selektivität der Bestimmung zu verbessern.

Es ergibt sich aus den obigen Beziehungen ferner, daß man ggf. auch so arbeiten kann, daß man mit einer markierten Chimäre (WT-A oder WT-B) arbeitet und nur die neutralen TNAb durch z.B. Vorinkubation mit Chimäre C (WT-C) der Messung entzieht.

**Modifizierung bekannter TBII-Assays durch Zusatz von TSHR-Chimären zur Gewinnung selektiverer Meßergebnisse (Verfahren gemäß den Ansprüchen 1 bis 5)**

Wie eingangs erläutert wurde, werden gegenwärtig zur Bestimmung von TSHR-Auto-Ab Assays genutzt, die auf einer Kompetition der in der Probe enthaltenen TSHR-Auto-Ab mit markiertem TSH um ein TSHR-Präparat, das auch festphasengebunden vorliegen kann (vgl. DYNOtest® TRAK), beruhen und die daher nur tatsächlich im o.g. Sinne mit TSH kompetierende TSHR-Auto-Ab (TBII) erfassen. TBII können nunmehr jedoch sowohl TSAb als auch TBAb sein bzw. umfassen. Arbeitet man bei einem der bekannten kompetitiven Assays wie oben beim Immunpräzipitationsassay mit einem Zusatz bzw. einer Vorinkubation mit einer geeigneten selektiven Chimäre in unmarkierter Form, kann eine Gruppe von TSHR-Auto-Ab der Kompetition und damit Messung entzogen werden, und es wird ein selektives Meßsignal erhalten, das im Idealfall nur für die TSAb bzw. TBAb in der Probe repräsentativ ist.

Die o.g. Erkenntnisse machen somit auch eine Erhöhung der Selektivität der bekannten kompetitiven TSHR-Auto-Ab-Assays möglich, was den Vorteil hat, daß alle gewohnten Arbeitsschritte, einschließlich einer Automatisierung, weiterhin genutzt werden können.

**Selektive TSHR-Auto-Ab-Bestimmungen nach anderen bekannten Assayprinzipien**

Das Prinzip der subtraktiven Neutralisierung von solchen TSHR-Auto-Ab-Typen, die bei einer Messung nicht erfaßt werden sollen, kann auch bei anderen an sich bekannten Assay-Designs zur Anwendung kommen. Erwähnt werden kann in diesem Zusammenhang die Beeinflussung der Bindungseffizienz von TSHR-Auto-Ab an eine im Überschuß vorhandene WT-TSHR-Festphase durch Zusatz selektiv bindender Chimären, wobei bei derartigen Assays die Markierung nach dem Bindungsschritt und einem Waschschritt auf unspezifische Weise z.B unter Verwendung von Potein A oder Protein G erfolgen kann. Ferner sind verschiendene an sich bekannte Formen von Sandwich-Assays zu erwähnen. Bei diesen werden z.B. wie oben die TSHR-Auto-Ab der Probe an eine Festphase gebunden, und unter Ausnutzung der Zweibindigkeit der TSHR-Auto-Ak wird zur Markierung statt mit Protein A oder G mit einem markierten WT-TSHR-Rezeptor oder einer markierten WT-Chimäre gearbeitet. Die Bindung des markierten Umsetzungspartners wird dabei durch Zusatz einer entsprechenden Chimäre im o.g. Sinne teilweise selektiv behindert, und es wird ein für einen TSHR-Auto-Ab-Typ selektives Meßsignal erhalten.

Weitere, nicht näher erläuterte Varianten derartiger Assays können sich dem Fachmann unter Berücksichtigung des oben erläuterten Prinzips der Differenzmessung unter Heranziehung der o.g. Beziehungen unmittelbar erschließen.

**Patentansprüche**

1. Verfahren zur differentialdiagnostischen Bestimmung von gegen den TSH-Rezeptor (TSHR) gebildeten Autoantikörpern (TSHR-Auto-Ab) in einer Serum- oder Plasmaprobe eines Patienten, der an einer Schilddrüsen-Autoimmunerkrankung leidet oder bei dem der Verdacht auf eine derartige Erkrankung besteht, **dadurch gekennzeichnet, daß** man

   in der Probe selektiv die Menge von wenigstens einem Typ von Autoantikörpern (TSHR-Auto-Ab), die ausgewählt sind aus (i) stimulierenden Autoantikörpern (TSAb) und/oder (ii) blockierenden Autoantikörpern (TBAb) und/oder (iii) weder stimulierend noch blockierend wirkenden, nicht pathogenen TSHR-Auto-Ab (TNAb) dadurch bestimmt, daß man die Probe mit einem gegebenenfalls markierten selektiven Bindungsreagens für alle oder mehrere Typen von TSHR-Auto-Ab, das auch den zu bestimmenden Typ von TSHR-Auto-Ab bindet, in Gegenwart eines Überschusses einer ausgewählten TSHR-Chimäre umsetzt, bei der die für die Bindung von stimulierenden und/oder blockierenden Autoantikörpern (TSAb und/oder TBAb) wesentlichen Sequenzen des TSHR durch entsprechende, keine Bindung des jeweiligen Typs von Autoantikörpern bewirkende Sequenzen ersetzt sind, und man dadurch die nicht zu bestimmenden TSHR-Auto-Ab-Typen, die von der ausgewählten TSHR-Chimäre gebunden werden, der Umsetzung mit dem Bindungsreagens entzieht,

   und man die Menge des ausgewählten zu bestimmenden Typs von TSHR-Auto-Ab anhand der Menge der an das Bindungsreagens gebundenen TSHR-Auto-Ab bestimmt.

2. Verfahren nach Anspruch 2, **dadurch gekennzeichnet, daß** man die Probe mit der ausgewählten TSHR-Chimäre vor der Umsetzung mit dem Bindungsreagens vorinkubiert oder Bindungsreagens und TSHR-Chimäre im wesentlichen gleichzeitig mit der Probe umsetzt.

**3.** Verfahren nach Anspruch 1 oder 2, **dadurch gekennzeichnet, daß** das Bindungsreagens ein solubilisierter direkt markierter vollständiger TSHR (WT-TSHR) ist und das Verfahren ein Immunpräzipitationsassay ist.

**4.** Verfahren nach Anspruch 1 oder 2, **dadurch gekennzeichnet, daß** das Bindungsreagens ein ggf. festphasengebundenes TSHR-Präparat eines herkömmlichen kompetitiven Assays zur Bestimmung von TSHR-Auto-Ab vom TBII-Typ ist.

**5.** Verfahren nach Anspruch 1 oder 2, **dadurch gekennzeichnet, daß** das Bindungsreagens festphasengebunden ist und im Überschuß vorliegt und der Assay vom Sandwich-Typ ist.

**Claims**

**1.** Method for the differential diagnostic determination of autoantibodies (TSHR-Auto-Ab) formed against the TSH receptor (TSHR) in a serum or plasma sample of a patient who is suffering from a thyroid autoimmune disease or who is suspected of having such a disease, **characterized in that**
the amount of at least one type of autoantibodies (TSHR-Auto-Ab) which is selected from (i) stimulating autoantibodies (TSAb) and/or (ii) blocking autoantibodies (TBAb) and/or (iii) neither stimulating nor blocking, nonpathogenic TSHR-Auto-Ab (TNAb) is determined selectively in a sample
by reacting the sample with an optionally labelled selective binding reagent for all or several types of TSHR-Auto-Ab which also binds to the type of TSHR-Auto-Ab to be determined, in the presence of an excess of a selected TSHR chimera in which the sequences of the TSHR which are essential for the binding of stimulating and/or blocking autoantibodies (TSAb and/or TBAb) have been replaced by corresponding sequences which do not affect any binding of the respective type of autoantibodies, and thus withdrawing the TSHR-Auto-Ab types not to be determined, which are bound by the selected TSHR chimera, from the reaction with the binding reagent,
and determining the amount of the selected type of TSHR-Auto-Ab to be determined on the basis of the amount of the TSHR-Auto-Ab bound to the binding reagent.

**2.** Method according to Claim 2, **characterized in that** the sample is preincubated with the selected TSHR chimera before the reaction with the binding reagent, or binding reagent and TSHR chimera are reacted substantially simultaneously with the sample.

**3.** Method according to Claim 1 or 2, **characterized in that** the binding reagent is a solubilized directly labelled complete TSHR (WT-TSHR) and the method is an immunoprecipitation assay.

**4.** Method according to Claim 1 or 2, **characterized in that** the binding reagent is an optionally solid phase-bound TSHR preparation of a conventional competitive assay for the determination of TSHR-Auto-Ab of the TBII type.

**5.** Method according to Claim 1 or 2, **characterized in that** the binding reagent is bound to the solid phase and is present in excess and the assay is of the sandwich type.

**Revendications**

**1.** Procédé pour déterminer, par diagnostic différentiel, la présence d'auto-anticorps formés contre le récepteur TSH (TSHR) dans un échantillon de sérum ou de plasma d'un patient, qui souffre d'une maladie autoimmune de la thyroïde ou pour lequel existe un soupçon d'une telle maladie, **caractérisé en ce que** l'on détermine sélectivement dans l'échantillon, la quantité d'au moins un type d'auto-anticorps (TSHR-auto-Ac), qui sont choisis parmi (i) les auto-anticorps stimulants (TSAc) et/ou (ii) les auto-anticorps bloquants (TBAc) et/ou (iii) les TSHR-auto-Ac ni stimulants, ni bloquants, ni pathogènes (TNAc), **en ce que** l'on fait réagir l'échantillon avec un réactif de liaison sélective, le cas échéant marqué, pour tous les types de TSHR-auto-Ac, qui se lie également au type à déterminer de TSHR-auto-Ac, en présence d'un excès d'un chimère choisi de TSHR, pour lequel les séquences essentielles pour la liaison des auto-anticorps stimulants et/ou bloquants (TSAc et/ou TBAc) du TSHR sont remplacées par des séquences correspondantes, n'effectuant aucune liaison du type particulier d'auto-anticorps, et on soustrait ainsi les types de TSHR-auto-Ac qui ne doivent pas être déterminés, qui sont liés aux chimères de TSHR choisis, de la réaction avec le réactif de liaison,

et **en ce que** l'on détermine la quantité du type à déterminer choisi de TSHR-auto-Ac à l'aide de la quantité de TSHR-auto-Ac lié au réactif de liaison.

2. Procédé selon la revendication 1, **caractérisé en ce que** l'on préincube l'échantillon avec les chimères de TSHR choisis avant la réaction avec le réactif de liaison ou l'on fait réagir le réactif de liaison et les chimères de TSHR essentiellement simultanément avec l'échantillon.

3. Procédé selon la revendication 1 ou 2, **caractérisé en ce que** le réactif de liaison est un TSHR complet, directement marqué, solubilisé (WT-TSHR) et **en ce que** le procédé est un dosage d'immunoprécipitation.

4. Procédé selon la revendication 1 ou 2, **caractérisé en ce que** le réactif de liaison est une préparation de TSHR le cas échéant liée à une phase solide d'un dosage compétitif usuel pour déterminer les TSHR-auto-Ac de type TBII.

5. Procédé selon la revendication 1 ou 2, **caractérisé en ce que** le réactif de liaison est lié à une phase solide et se présente en excès et le dosage est de type sandwiche.

# Fig. 1

# Fig. 2

# Fig. 3

Fig. 4

# Fig. 5

**Fig. 6**